(19) Europäisches Patentamt
European Patent Office
Office européen des brevets

(11) Veröffentlichungsnummer : **0 388 359 B1**

# (12) EUROPÄISCHE PATENTSCHRIFT

(45) Veröffentlichungstag der Patentschrift : **12.05.93 Patentblatt 93/19**

(51) Int. Cl.$^5$ : **C07D 233/22,** C08G 59/50

(21) Anmeldenummer : **90810183.5**

(22) Anmeldetag : **08.03.90**

(54) **Imidazolinverbindungen.**

(30) Priorität : **17.03.89 GB 8906198**

(43) Veröffentlichungstag der Anmeldung : **19.09.90 Patentblatt 90/38**

(45) Bekanntmachung des Hinweises auf die Patenterteilung : **12.05.93 Patentblatt 93/19**

(84) Benannte Vertragsstaaten : **AT CH DE ES FR GB IT LI NL SE**

(56) Entgegenhaltungen : **US-A- 2 888 458**

(73) Patentinhaber : **CIBA-GEIGY AG**
**Klybeckstrasse 141**
**CH-4002 Basel (CH)**

(72) Erfinder : **Bagga, Madan Mohan, Dr.**
**56 Hurrell Road**
**Cambridge CB4 3RH (GB)**

(74) Vertreter : **Sharman, Thomas et al**
**CIBA-GEIGY PLC. Patent Department, Central Research, Hulley Road**
**Macclesfield, Cheshire SK10 2NX (GB)**

EP 0 388 359 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft substituierte Imidazolinverbindungen, die als Beschleuniger in härtbaren Epoxidharzgemischen verwendet werden können, die als Klebstoffe oder Dichtungsmassen geeignet sind.

Die Verwendung von Epoxidharzen in Klebstoffen und Dichtungsmassen wird seit Jahrzehnten kommerziell durchgeführt. Da viele Härtungsmittel für Epoxidharze bei Raumtemperatur reagieren, können diese mit dem Epoxidharz erst kurz vor dem Gebrauch gemischt werden. Andere Härtungsmittel sind mit Epoxidharzen bei Raumtemperatur stabil und härten erst bei erhöhter Temperatur. Solche Härtungsmittel, die als latente Härtungsmittel bezeichnet werden, sind im Handel erhältlich und schliessen Dicyandiamid und Polycarbonsäurehydrazide ein.

Gemische enthaltend ein Epoxidharz und einen solchen latenten Härter benötigen zum Härten bei 180°C etwa 15 Minuten bis 1 Stunde. Die Härtungszeit kann durch Zugabe eines Beschleunigers verkürzt werden. Ein Beschleuniger, der oft verwendet wird, wenn die Epoxidharzgemische Formstoffe mit guter Schlagfestigkeit und Hitzebeständigkeit ergeben sollen, ist Benzimidazol. Doch weisen Epoxidharzgemische, die Benzimidazol als Beschleuniger enthalten, eine unerwünscht beschränkte Lagerstabilität bei Raumtemperatur auf.

Es wurde nun gefunden, dass neue, substituierte Imidazolinverbindungen, die durch Umsetzung eines Esters einer aromatischen Hydroxysäure mit Diethylentriamin oder N-(2-Aminoethyl)-1,3-propandiamin erhalten werden, geeignete Beschleuniger für Epoxidharzgemische darstellen, die Dicyandiamid und Polycarbonsäurehydrazide als latente Härtungsmittel enthalten. Solche Gemische weisen eine verlängerte Lagerstabilität bei Raumtemperatur auf und härten schnell bei erhöhter Temperatur aus. Daraus hergestellte Formstoffe zeichnen sich ausserdem durch eine gute Schlagfestigkeit und Hitzebeständigkeit aus.

Gegenstand vorliegender Erfindung sind somit Imidazolinverbindungen der Formel I

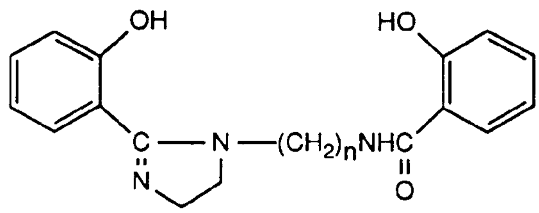

(I),

worin n für 2 oder 3 steht.

Die Imidazolinverbindungen der Formel I können durch Erhitzen von Methylsalicylat mit einem Amin der Formel II

$$NH_2CH_2CH_2NH(CH_2)_nNH_2 \qquad (II),$$

worin n die gleiche Bedeutung wie in Formel I hat, hergestellt werden, wobei erst eine Verbindung der Formel III erhalten wird

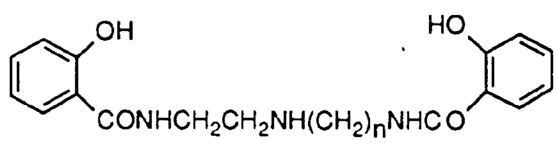

(III),

die durch Erhitzen auf höhere Temperaturen unter Ausscheidung von Wasser und unter Ringschluss zur Imidazolinverbindung der Formel I umgewandelt wird. Das Molverhältnis von Methylsalicylat zu Amin beträgt im allgemeinen wenigstens 2:1 und ist vorzugsweise 3:1 bis 4:1.

Die Herstellung kann mit und ohne Isolation des Zwischenproduktes vorgenommen werden. Beispielsweise können Diethylentriamin und der Methylester der Salicylsäure auf 80 bis 110°C erhitzt werden, wobei eine feste Verbindung der Formel III erhalten wird, die durch Filtration aus dem Reaktionsgemisch isoliert wird, gegebenenfalls unter Zugabe eines Verdünners, der die feste Substanz nicht löst, wie beispielsweise Aceton, zum Reaktionsgemisch. Nach dem Trocknen wird die feste Substanz unter Vakuum auf 140-200°C erhitzt, wobei der Ringschluss einsetzt.

Nach einer anderen Methode werden das Diethylentriamin und der Methylester der Salicylsäure bei 80 bis 110°C umgesetzt, wobei das bei der Reaktion entstehende Methanol abdestilliert wird. Die restliche Mischung wird dann auf 140-200°C erhitzt, wobei Ringschluss eintritt. Bei dieser Methode können das Triamin und der Ester in einem Lösungsmittel mit einem Siedepunkt über 110°C gelöst sein, beispielsweise in Xylol

oder Ethylenglykol, wobei die Ringschlussbildung durch azeotropes Entfernen von Wasser aus dem Reaktionsgemisch beschleunigt werden kann. Andererseits kann das Reaktionsgemisch auch unter Rückfluss gehalten werden, wobei Anfang- und Endreaktion in einer Stufe ablaufen.

Gegenstand der vorliegenden Erfindung ist auch ein härtbares Gemisch, enthaltend

(A) ein Epoxidharz

(B) als latentes Härtungsmittel für (A) Dicyandiamid oder ein Hydrazid einer Polycarbonsäure und

(C) als Härtungsbeschleuniger und dispergiert als Pulver im Gemisch eine Imidazolinverbindung der Formel I.

Geeignete Epoxidharze (A) sind beispielsweise Harze mit durchschnittlich mehr als einer Epoxidgruppe im Molekül, einschliesslich solcher, die durchschnittlich mehr als eine Glycidylgruppe aufweisen, welche direkt an ein Sauestoff-, Stickstoff- oder Schwefelatom gebunden sind.

Als Beispiele solcher Harze können die Polyglycidylester genannt werden, die durch Umsetzung einer Verbindung mit zwei oder mehreren Carboxylgruppen pro Molekül und Epichlorhydrin, Glycerindichlorhydrin oder β-Methylepichlorhydrin in Gegenwart von Alkali erhalten werden. Solche Polyglycidylester können sich von aliphatischen Carbonsäuren, wie beispielsweise Oxalsäure, Bernsteinsäure, Glutarsäure, Adipinsäure, Pimalinsäure, Suberinsäure, Azelainsäure, Sebacinsäure oder dimerisierter oder trimerisierter Linolsäure, cycloaliphatischen Polycarbonsäuren, wie Tetrahydrophthalsäure, 4-Methyltetrahydrophthalsäure, Hexahydrophthalsäure und 4-Methylhexahydrophthalsäure, und von aromatischen Polycarbonsäuren, wie Phthalsäure, Isophthalsäure und Terephthalsäure, ableiten.

Weitere Beispiele sind Polyglycidylether, die durch Umsetzung von Verbindungen mit wenigstens zwei alkoholischen oder phenolischen Hydroxylgruppen mit dem entsprechenden Epichlorhydrin unter alkalischen Bedingungen oder in Gegenwart eines sauren Katalysators mit alkalischer Nachbehandlung erhalten werden. Solche Ether können sich von acyclischen Alkoholen ableiten, wie Ethylenglykol, Diethylenglykol und höhere Poly-(oxyethylen)-glykole, Propan-1,2-diol und Poly-(oxypropylen)-glykole, Propan-1,3-diol, Butan-1,4-diol, Poly-(oxytetramehtylen)-glykole, Pentan-1,5-diol, Hexan-1,6-diol, Hexan-2,4,6-triol, Glycerin, 1,1,1-Trimethylolpropane, Pentaerythrit und Sorbit, von cycloaliphatischen Alkohlen, wie Resorcitol, Chinitol, Bis-(4-hydroxycyclohexyl)-methan, 2,2-Bis-(4-hydroxycyclohexyl)-propan, und 1,1-Bis-(4-hydroxymethyl)-cyclohex-3-en, von Alkoholen mit einem aromatischen Kern, wie N,N-Bis-(2-hydroxyethyl)-anilin und von im US-Patent 4 284 574 beschriebenen Alkoholen, wie 2,2-Bis-(p-(3-ethoxy-2-hydroxypropyloxy)phenyl)-propan, 2,2-Bis-(p-(3-butoxy-2-hydroxypropyloxy)-phenyl)-propan oder Bis-(p-(3-butoxy-2-hydroxypropyloxy)-phenyl)-sulphon. Sie können sich auch von einkernigen Phenolen, wie Resorcin oder Hydrochinon, und von mehrkernigen Phenolen, wie Bis-(4-hydroxyphenyl)-methan, 4,4′-Dihydroxydiphenyl, Bis-(4-hydroxyphenyl)-sulfon, 1,1,2,2-Tetrakis-(4-hydroxyphenyl)-ethan, 2,2-Bis-(4-hydroxyphenyl)-propan, 2,2-Bis(3,5-dibromo-4-hydroxyphenyl)-propan, und von Novolaken ableiten, hergestellt aus Aldehyden, wie Formaldehyd, Acetaldehyd, Chloral oder Furfural mit Phenolen, wie Phenol selbst oder mit am Ring durch Chloratome oder Alkylgruppen substituierten Phenolen mit bis zu 9 C-Atomen in der Alkylgruppe, wie 4-Chlorphenol, 2-Methylphenol oder 4-tert.-Butylphenol.

Poly-(N-glycidyl)-verbindungen, beispielsweise solche, die durch Dehydrochlorierung von Reaktionsprodukten aus Epichlorhydrin mit Aminen erhalten werden, welche mindestens zwei Aminwasserstoffatome enthalten, wie Bis-(4-methylaminophenyl)-methan, Triglycidylisocyanurat, und N,N'-Diglycidylderivate von cyclischen Harnstoffen, wie Ethylenharnstoff oder 1,3-Propylenharnstoff, oder von einem Hydantoin, wie 5,5-Dimethylhydantoin.

Beispiele für Poly-(S-glycidyl)-verbindungen sind die Di-S-glycidylderivate von Dithiolen, wie Ethan-1,2-dithiol oder Bis-(4-mercaptomethylphenyl)-ether.

Epoxidharze mit Glycidylgruppen an verschiedenen Heteroatomen können ebenfalls verwendet werden, wie N,N,O-Triglycidylderivat von 4-Aminophenol, der Glycidyletherglycidylester von Salicylsäure, N-Glycidyl-N'-(2-glycidyloxypropyl)-5,5-dimethylhydantoin, und 2-Glycidyloxy-1,3-bis-(5,5-dimethyl-1-glycidylhydantoin-3-yl)-propan.

Gewünschtenfalls können auch Gemische von Epoxidharzen eingesetzt werden.

Bevorzugt werden flüssige Epoxidharze von Polyglycidylethern, Polyglycidylestern, N,N-Diglycidylhydantoinen und Poly-(N-glycidyl)-derivate aromatischer Amine eingesetzt.
Besonders bevorzugte Harze sind Polyglycidylether von Bisphenolen, besonders 2,2-Bis-(4-hydroxyphenyl)-propan (Bisphenol A), und Mischungen davon mit Polyglycidylethern mehrwertiger Alkohole, besonders von Butane-1,4-diol.

Das latente Härtungsmittel (B) kann Dicyandiamid oder ein Hydrazid einer Polycarbonsäure sein. Geeignete Hydrazide sind die von aliphatischen und aromatischen Dicarbonsäuren, wie Stearinsäure-, Adipinsäure- oder Isophthalsäuredihydrazid, wobei die letzten beiden bevorzugt sind.

Die Menge des latenten Härtungsmittels (B) im erfindungsgemässen Gemisch entspricht der üblichen Menge des betreffenden Härtungsmittels bezogen auf das Epoxidharz. Solche Mengen sind dem Fachmann

für Epoxidharzformulierungen bekannt. Wenn (B) Dicyandiamid ist, beträgt die Menge im allgemeinen 1 bis 30, vorzugsweise 3 bis 20, insbesondere 5 bis 10 Teile pro 100 Teile Epoxidharz (A). Ist (B) ein Hydrazid einer Polycarbonsäure, so wird die Menge im allgemeinen so gewählt, dass pro Epoxidäquivalent des Harzes (A) 0,5 bis 1,5, vorzugsweise 0,8 bis 1,2, insbesondere 0,9 bis 1,1 aktive Aminwasserstoffäquivalente des Hydrazids vorliegen.

Die Menge der Imidazolin-Verbindung als Beschleuniger (C) ist nicht kritisch, vorausgesetzt eine wirksame Menge für den Beschleunigungseffekt ist vorhanden. Im allgemeinen werden Mengen im Bereich von 0,1 bis 20 Gew.-%, vorzugsweise von 1 bis 10 Gew.-% und insbesondere von 2 bis 5 Gew.-%, bezogen auf das Epoxidharz (A), eingesetzt.

Der Imidazolin-Beschleuniger (C) wird im allgemeinen zu einem feinen Pulver gemahlen, beispielsweise zu einem Pulver mit einem Partikeldurchmesser von 0,15 mm, bevor er mit den anderen Bestandteilen des Gemisches vermischt wird. Gröbere Partikel der Imidazoline (C) können gewöhnlich dem Gemisch zugesetzt werden, da das Mischen der Mischungskomponenten in konventionellen Mischungsapparaturen, wie beispielsweise Kugelmühle, vorgenommen wird, wobei die Partikel zerkleiner werden können.

Die erfindungsgemässen Gemische können ferner Additive enthalten, die üblicherweise Epoxidharzgemischen zugesetzt werden, um deren physikalischen oder chemischen Eigenschaften vor oder nach der Härtung zu verbessern. Solche Zusätze sind beispielsweise Pigmente, Farbstoffe, Flexibilisatoren, Plastifizierungsmittel, Füllstoffe, Thioxotropiermittel und Flammschutzmittel. Geeignetes polymeres Material, welches als Schlagfestmacher zugesetzt werden kann, sind Acrylatester von Epoxidharzen, Polyurethan-Prepolymere, verkappte Polyisocyanate und elastomere Butadienpolymere.

Die erfindungsgemässen Imidazolinverbindungen sind besonders geeignet für Gemische, die ein elastomeres Butadienpolymer als Schlagfestmacher enthalten. Solche Polymere schliessen elastomere Copolymere von Butadien mit Acrylnitril ein, insbesondere solche Copolymere, die funktionelle Gruppen aufweisen, die mit Epoxidgruppen reagieren, wie Hydroxyl-, Amin- und insbesondere Carboxylgruppen. Reaktive, funktionelle Gruppen aufweisende Copolymere dieser Art sind im Handel von der Firma Goodrich unter der Bezeichnung HYCAR® erhältlich.

Diese reaktive Gruppen aufweisende Copolymere werden den erfindungsgemässen Gemischen vorzugsweise als vorgeformte Addukte mit Epoxidharzen zugesetzt. Bevorzugte Addukte sind die epoxidgruppenterminierten Addukte eines carboxylgruppenterminierten Acrylnitril-Butadien-Copolymers mit einem Polyglycidylether eines Bisphenols, vorzugsweise Bisphenol A. Das schlagfestmachende Polymer kann in Mengen bis zu 100 Gew.-%, bezogen auf das Epoxidharz (A), dem Gemisch zugesetzt werden.

Wie bereits erwähnt, sind die bevorzugt verwendeten Epoxidharze (A) flüssige Harze. Die härtbaren Gemische, die solche Harze enthalten, können ungefüllte Gemische niedriger Viskosität bis pasten- oder kittartige Gemische mit hohem Anteil an Füllstoffen oder Additiven sein. Die erfindungsgemässen Gemische können auch als Filme oder Schichten vorliegen, die gegebenenfalls faserverstärkt sind oder durch eine Glasfasermasse verstärkt sind.

Die erfindungsgemässen Gemische können bei erhöhter Temperatur ausgehärtet werden, im allgemeinen zwischen 140 bis 220°C, vorzugsweise zwischen 160 bis 200°C. Die Härtung kann in weniger als einer Minute vorgenommen werden, besonders bei höheren Temperaturen, doch kann das Erhitzen auch fortgesetzt werden, beispielsweise bis zu 3 Stunden, um die physikalischen Eigenschaften des gehärteten Produktes zu verbessern. Ist eine schnelle Härtung erforderlich, beispielsweise beim Verbinden und Verkitten von Automobilteilen, kann dies in bekannter Weise durch Induktionsheizung vorgenommen werden.

Die härtbaren Gemische können als Beschichtungsmassen, als Giess- oder Laminierharze oder vorzugsweise als Klebstoffe oder Dichtungsmassen verwendet werden.

Erfindungsgegenstand ist somit auch die Verwendung der erfindungsgemässen Gemische zum Verkleben oder Versiegeln von zwei Oberflächen untereinander, wobei das härtbare Gemisch auf eine oder beide Oberflächen aufgetragen wird, die dann so zusammengelegt werden, dass sich das härtbare Gemisch zwischen beiden Oberflächen befindet. Anschliessend wird das Gebilde gehärtet. Auf diese Weise können Oberflächen aus Metall, wie Stahl oder Aluminium, Plastik, Glas, Reibungsmaterial, wie Bremsbelege, oder keramischem Material verbunden werden. Besonders geeignet ist diese Methode wenn beide Oberflächen aus Metall sind.

Die Erfindung wird durch die folgenden Beispiele erläutert, worin Teile Gewichtsteile und Prozente Gewichtsprozente bedeuten.

HYCAR CTBN 1300X13, das in den Beispielen verwendet wird, ist ein carboxylterminiertes Copolymer aus Acrylnitril und Butadien und von der Firma Goodrich, USA, im Handel erhältlich.

<u>Beispiel 1:</u> 22,97 g (0,22 Mol) Diethylentriamin werden unter Rühren tropfenweise zu 102,6 g (0,68 Mol) Methylsalicylat zugegeben. Das erhaltene Gemisch wird unter Rückfluss 6 Stunden erhitzt, wobei ein weisser Niederschlag ausfällt. Dann wird das Reaktionsgemisch auf 30°C abgekühlt. Nach Zugabe von Aceton wird

der weisse Niederschlag abfiltriert, mit Aceton gewaschen und im Vakuumofen bei 60°C getrocknet. Gemäss Elementar- und Spektralanalyse entspricht das erhaltene Zwischenprodukt, das einen Schmelzpunkt von 150-152°C aufweist, einer Verbindung der Formel III, worin n für 2 steht.

Elementaranalyse:

Gefunden: C=63,48%; H=6,16%, N=12,39%
Theorie: C=62,95%; H=6,16%; N=12,24%.

Das Zwischenprodukt wird in einem Vakuumofen unter einem Vakuum der Wasserstrahlpumpe während 90 Minuten auf 160°C erhitzt, zu einem Pulver zerkleinert und dann wie zuvor für eine weitere Stunde im Vakuum auf 160°C erhitzt. Gemäss Elementar- und Spektralanalyse entspricht die erhaltene Imidazolinverbindung der Formel I, worin n für 2 steht.

Elementaranalyse:

Gefunden: C=66,18%; H=5,96%; N=12,87%
Theorie: C=66,46%; H=5,85%; N=12,92%.

$^1$H-NMR-Spektrum (Essigsäure $d_4$): 6.6-7.7(m-8H), 4.3 (t-2H), 4.1 (t-2H), 3.7(s-4H)$\delta$.

Beispiel 2: 25,75 g (0,25 Mol) Diethylentriamin werden unter Rühren tropfenweise zu 152 g (1 Mol) Methylsalicylat zugegeben. Das Gemisch wird unter Rückfluss 2 Stunden erhitzt, wobei die Temperatur von anfangs 105°C auf 90°C zurückgeht und ein Niederschlag ausfällt. Das bei der Umsetzung entstehende Methanol wird bei Normaldruck abdestilliert, und dann wird das Reaktionsgemisch für 2 Stunden auf 170°C erhitzt. Das Reaktionsgemisch wird für weitere 30 Minuten auf 170°C unter vermindertem Druck von 533 mbar erhitzt und dann auf 30°C abgekühlt. Zu der erhaltenen viskosen Masse wird Aceton als Verdünner zugegeben, und der erhaltene weisse Niederschlag wird abfiltriert, mit Aceton gewaschen und im Vakuumofen bei 60°C getrocknet. Es werden 31 g eines Produktes erhalten, das gemäss Elementar- und Spektralanalyse dem des Beispiels 1 entspricht.

Beispiel 3: 11,7 g (0,10 Mol) N-(2-Aminoethyl)-1,3-propandiamin werden tropfenweise zu einer Lösung von 30,4 g (0,20 Mol) Methylsalicylat in 100 ml Ethylenglykol zugegeben und dann während 4 Stunden am Rückfluss erhitzt, wobei ein weisser Niederschlag ausfällt. Die Mischung wird dann auf 30°C gekühlt, filtriert, der Rückstand mit Aceton gewaschen und im Vakuumofen getrocknet. Unter diesen Bedingungen wird die Umsetzung bis zur Ringschliessung durchgeführt. Es werden 13,8 g des Endproduktes mit einem Schmelzpunkt von 280-282°C erhalten. Gemäss Elementar- und Spektralanalyse entspricht die erhaltene Imidazolinverbindung der Formel I, worin n für 3 steht.

Elementaranalyse:

Gefunden: C=66,27%; H=6,31%; N=12,26%
Berechnet: C=66,46%; H=5,85%; N=12,92%.

Beispiele 4-6: Härtbare, pastenartige Zusammensetzungen werden durch Dispergieren eines pulvrigen Härters und von 2 Teilen der Imidazolinverbindung von Beispiel 1 zusammen mit 5 Teilen hochdisperser Kieselsäure als Füllstoff in 100 Teilen eines flüssigen Bisphenol A-diglycidylethers mit einem Epoxidgehalt von 5,2 Aequivalenten/kg hergestellt. Die Gelierungszeiten der Zusammensetzungen bei bestimmten Temperaturen werden gemessen, indem Proben auf eine bis zur Testtemperatur erhitzte Heizplatte aufgebracht werden und deren Gelierzeiten ermittelt werden. Die Lagerstabilitäten der Zusammensetzungen werden ermittelt durch Lagern dieser in Tuben in einem auf 40°C erwärmten Ventilationsofen, und das Ende der Lagerstabilität tirtt dann ein, wenn die Zusammensetzung nicht mehr streichfähig ist.

Der betreffende Härter und dessen Menge in den Zusammensetzungen, sowie die Gelierzeiten und Lagerstabilitäten der Zusammensetzungen werden in der folgenden Tabelle angegeben.

| Beispiel | Härter | Menge (Teile) | Gelierzeit (Min.) 160°C | 180°C | Lagerstabilität |
|---|---|---|---|---|---|
| 4 | Dicyandiamid | 7,5 | 5,07 | 1,25 | über 5 Wochen |
| 5 | Adipinsäure-dihydrazid | 23,1 | 6,33 | 0,93 | über 5 Wochen |
| 6 | Isophthalsäure-dihydrazid | 25,2 | 5,15 | 1,32 | über 5 Wochen |

Beispiel 7: Die Arbeitsweise von Beispielen 4-6 wird wiederholt, wobei anstelle von 100 Teilen des flüssigen Bisphenol A-diglycidylethers nun ein Gemisch aus 80 Teilen des flüssigen Bisphenol A-diglycidylethers zusammen mit 20 Teilen eines flüssigen Butandiol-1,4-diglycidylethers mit einem Epoxidgehalt von 8,8 Aequivalenten/kg verwendet wird. Dazu werden 8 Teile Dicyandiamid und 4 Teile Kieselsäure zugemischt. Man erhält folgendes Ergebnis:

| Gelierzeit (Min.) 160°C | 180°C | Lagerstabilität |
|---|---|---|
| 2,31 | 0,68 | über 15 Wochen |

Beispiel 8: Die Arbeitsweise von Beispiel 7 wird wiederholt, wobei das Imidazol von Beispiel 3 anstelle von Beispiel 1 verwendet wird. Man erhält folgendes Resultat.

| Gelierzeit (Min.) 160°C | 180°C | Lagerstabilität |
|---|---|---|
| 2,98 | 0,75 | über 15 Wochen |

Beispiel 9: Eine Klebstoffmischung wird aus folgenden Komponenten hergestellt.

| | |
|---|---|
| Epoxidharz | 100 Teile |
| Dicyandiamid | 7,5 Teile |
| hochdisperse Kieselsäure | 5 Teile |
| Mikroglaskugeln | 1 Teile |
| Imidazolin der Formel I, worin n = 2 ist | 2 Teile |

Das verwendete Epoxidharz ist ein Diglycidylether des Bisphenol A mit einem Epoxidgehalt von 5,2 Aequivalenten/kg. Mikroglaskugeln werden zur Kontrolle der Klebflächendicke hinzugegeben.

Die Zusammensetzung wird auf entfettete, sandgestrahlte Stahlbleche aufgetragen, die so verklebt werden, dass Ueberlappungsbereiche von 645 $mm^2$ entstehen. Die Härtung wird während 15 Minuten bei 180°C

vorgenommen. Dann werden die Ueberlappungsstellen auf Raumtemperatur abgekühlt. Die Ueberlappungsscherfestigkeit, gemessen mit einer Zuggeschwindigkeit von 7,5 mm/Min., beträgt 14,15 MPa.

Beispiel 10: Die Arbeitsweise von Beispiel 9 wird wiederholt, wobei die Zusammensetzung durch folgende Klebstoffmischung ersetzt wird.

| | |
|---|---|
| Epoxidharz gemäss Beispiel 9 | 80 Teile |
| Butan-1,4-dioldiglycidylether (Epoxidgehalt 8,8 Aequiv./kg) | 20 Teile |
| Dicyandiamid | 8 Teile |
| Hochdisperse Kieselsäure | 5 Teile |
| Mikroglaskugeln | 1 Teil |
| Imidazolin gemäss Beispiel 1 | 2 Teile |

Die Ueberlappungsscherfestigkeit beträgt 15,34 MPa.

Beispiel 11: Beispiel 9 wird wiederholt, wobei Dicyandiamid durch 23,1 Teile Adipinsäurehydrazid ersetzt wird. Die Ueberlappungsscherfestigkeit beträgt 11,29 MPa.

Beispiel 12: Beispiel 9 wird wiederholt, wobei Dicyandiamid durch 25,2 Teile Isophthalsäuredihydrazide ersetzt wird. Die Ueberlappungsscherfestigkeit beträgt 10,76 MPa.

Beispiel 13: Eine Klebstoffpaste wird hergestellt, indem man 9,5 Teile Dicyandiamid, 1,8 Teile des Imidazolins gemäss Beispiel 1 und 6 Teile hochdisperse Kieselsäure als Füllstoff in eine Mischung aus 80 Teilen eines flüssigen Bisphenol A-diglycidylethers mit einem Epoxidgehalt von 5,4 Aequivalenten/kg und 35 Teilen eines Adduktes aus diesem Diglycidylether mit gleichen Mengen HYCAR CTBN 1300x13 (hergestellt durch Erhitzen der Bestandteile während 2 Stunden auf 150°C) mit einem Epoxidgehalt von 2,4 Aequivalenten/kg dispergiert. Von dieser Paste wird ein dünner Film auf eine mit einem Trennmittel beschichtete Zinnfolie gesprüht. Der Film wird durch Erhitzen während 15 Minuten auf 190°C gehärtet und dann für thermomechanische Untersuchungen abgezogen. In einem thermomechanischen Testgerät von Perkin-Elmer, Modell TMS 2, wird eine gehärtete Probe von 0,62 mm Dicke unter einem Belastungsgewicht von 100 g und einer Aufheizungsrate von 10°C/Min auf deren Eindringungsfestigkeit getestet. Das Eindringen in die Probe setzte bei 130,6°C ein.

## Patentansprüche

### Patentansprüche für folgende Vertragsstaaten : AT, CH, DE, FR, GB, IT, LI, NL, SE

1. Imidazolinverbindungen der Formel I

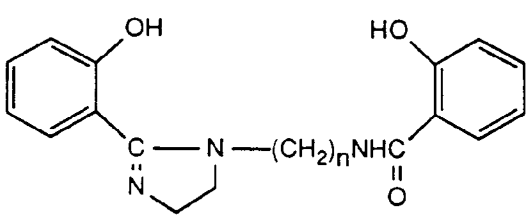

(I),

worin n für 2 oder 3 steht.

2. Verfahren zur Herstellung von Imidazolinverbindungen der Formel I gemäss Anspruch 1, dadurch gekennzeichnet, dass man Methylsalicylat mit einem Amin der Formel II

$$NH_2CH_2CH_2NH(CH_2)_nNH_2 \quad (II),$$

worin n für 2 oder 3 steht, erhitzt, wobei eine Verbindung der Formel III erhalten wird

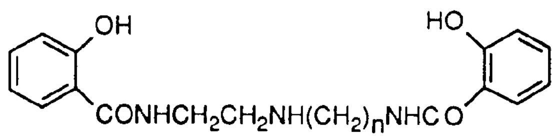

(III),

und dann die Verbindung der Formel III auf eine höhere Temperatur erhitzt, wobei unter Ausscheidung von Wasser und unter Ringschluss eine Imidazolinverbindung gemäss Formel I erhalten wird.

3. Verfahren zur Herstellung von Imidazolinverbindungen gemäss Anspruch 2, dadurch gekennzeichnet, dass man Methylsalicylat und ein Amin der Formel II

$$NH_2CH_2CH_2NH(CH_2)_nNH_2 \qquad (II),$$

in einem Lösungsmittel unter Rückfluss so lange erhitzt, bis die Imidazolinverbindung entstanden ist.

4. Verfahren gemäss Anspruch 2 oder 3, dadurch gekennzeichnet, dass ein Molverhältnis von Methylsalicylat zum Amin von 3:1 bis 4:1 verwendet wird.

5. Härtbares Gemisch, enthaltend (A) ein Epoxidharz und (B) als latentes Härtungsmittel für (A) Dicyandiamid oder ein Hydrazid einer Polycarbonsäure und (C) als Härtungsbeschleuniger, dispergiert als Pulver im Gemisch, eine Imidazolinverbindung gemäss Anspruch 1.

6. Gemisch gemäss Anspruch 5, enthaltend ein Epoxidharz (A) mit durchschnittlich mehr als einer Glycidylgruppe im Molekül, welche an ein Sauerstoff-, Stickstoff- oder Schwefelatom gebunden ist.

7. Gemisch gemäss Anspruch 6, worin das Epoxidharz (A) flüssig und ein Polyglycidylether, ein Polyglycidylester, ein N,N'-Diglycidylhydantoin oder eine Poly(N-glycidyl)-Verbindung eines aromatischen Amins ist.

8. Gemisch gemäss Anspruch 5, worin der Härtungsbeschleuniger (C) in Mengen von 0,1 bis 20 Gew.-%, bezogen auf das Epoxidharz (A), vorliegt.

9. Gemisch gemäss Anspruch 5, worin der Härtungsbeschleuniger (C) in Mengen von 1 bis 10 Gew.-%, bezogen auf das Epoxidharz (A), vorliegt.

10. Gemisch gemäss Anspruch 5, enthaltend ausserdem ein elastomeres Dienpolymerisat als Schlagfestmacher.

11. Verwendung des härtbaren Gemisches gemäss Anspruch 5 zum Verkleben oder Versiegeln von zwei Oberflächen miteinander, insbesondere solchen aus Metall.

**Patentansprüche für folgenden Vertragsstaat : ES**

1. Verfahren zur Herstellung von Imidazolinverbindungen der Formel I

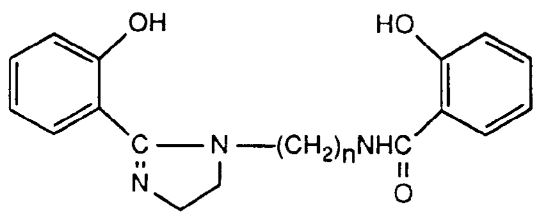

(I),

worin n für 2 oder 3 steht, dadurch gekennzeichnet, dass man Methylsalicylat mit einem Amin der Formel II

$$NH_2CH_2CH_2NH(CH_2)_nNH_2 \qquad (II),$$

worin n für 2 oder 3 steht, erhitzt, wobei eine Verbindung der Formel III erhalten wird

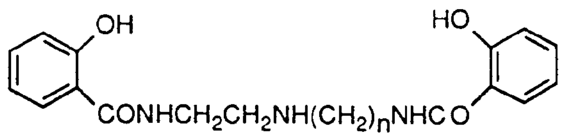

(III),

und dann die Verbindung der Formel III auf eine höhere Temperatur erhitzt, wobei unter Ausscheidung von Wasser und unter Ringschluss eine Imidazolinverbindung gemäss Formel I erhalten wird.

2. Verfahren zur Herstellung von Imidazolinverbindungen gemäss Anspruch 1, dadurch gekennzeichnet, dass man Methylsalicylat und ein Amin der Formel II

$$NH_2CH_2CH_2NH(CH_2)_nNH_2 \qquad (II),$$

in einem Lösungsmittel unter Rückfluss so lange erhitzt, bis die Imidazolinverbindung entstanden ist.

3. Verfahren gemäss Anspruch 1 oder 2, dadurch gekennzeichnet, dass ein Molverhältnis von Methylsalicylat zum Amin von 3:1 bis 4:1 verwendet wird.

## Claims

**Claims for the following Contracting States : AT, CH, DE, FR, GB, IT, LI, NI, SE**

1. Imidazoline compounds of the formula I

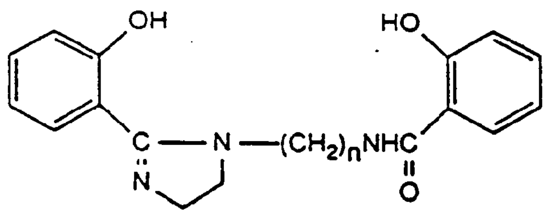

(I),

wherein n represents 2 or 3.

2. Process for the preparation of imidazoline compounds of the formula I according to claim 1, characterised in that methyl salicylate is heated with an amine of the formula II

$$NH_2CH_2CH_2NH(CH_2)_nNH_2 \qquad (II),$$

wherein n represents 2 or 3, a compound of the formula III

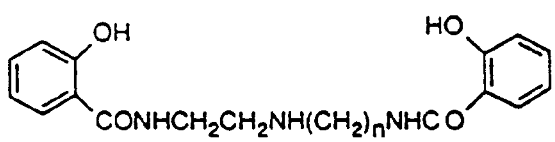

(III),

being obtained, and the compound of the formula III is then heated to a higher temperature, an imidazoline compound according to formula I being obtained with elimination of water and with cyclization.

3. Process for the preparation of imidazoline compounds according to claim 2, characterised in that methyl salicylate and an amine of the formula II

$$NH_2CH_2CH_2NH(CH_2)_nNH_2 \qquad (II),$$

are heated under reflux in a solvent until the imidazoline compound has formed.

4. Process according to claim 2 or 3, characterised in that a molar ratio of methyl salicylate to amine of 3:1 to 4:1 is used.

5. Curable mixture comprising (A) an epoxy resin and (B) dicyandiamide or a hydrazide of a polycarboxylic

acid, as a latent curing agent for (A), and (C) an imidazoline compound according to claim 1, dispersed as a powder in the mixture, as a curing accelerator.

**6.** Mixture according to claim 5, comprising an epoxy resin (A) having on average more than one glycidyl group, bonded to an oxygen, nitrogen or sulfur atom, in the molecule.

**7.** Mixture according to claim 6, wherein the epoxy resin (A) is liquid and is a polyglycidyl ether, a polyglycidyl ester, an N,N'-diglycidylhydantoin or a poly(N-glycidyl) compound of an aromatic amine.

**8.** Mixture according to claim 5, wherein the curing accelerator (C) is present in amounts of 0.1 to 20 % by weight, based on the epoxy resin (A).

**9.** Mixture according to claim 5, wherein the curing accelerator (C) is present in amounts of 1 to 10 % by weight, based on the epoxy resin (A).

**10.** Mixture according to claim 5, furthermore comprising an elastomeric diene polymer as an impact modifier.

**11.** Use of the curable mixture according to claim 5 for glueing or sealing two surfaces to one another, in particular those of metal.

**Claims for the following Contracting State : ES**

**1.** Process for the preparation of imidazoline compounds of the formula I

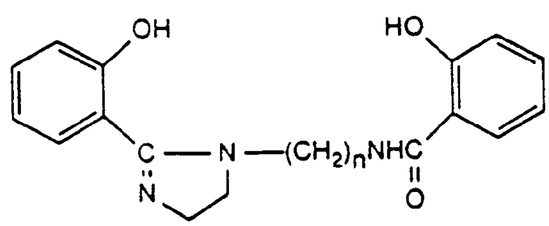

(I),

wherein n represents 2 or 3, characterised in that methyl salicylate is heated with an amine of the formula II

$$NH_2CH_2CH_2NH(CH_2)_nNH_2 \quad (II),$$

wherein n represents 2 or 3, a compound of the formula III

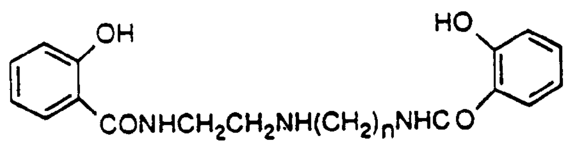

(III),

being obtained, and the compound of the formula III is then heated to a higher temperature, an imidazoline compound according to formula I being obtained with elimination of water and with cyclization.

**2.** Process for the preparation of imidazoline compounds according to claim 1, characterised in that methyl salicylate and an amine of the formula II

$$NH_2CH_2CH_2NH(CH_2)_nNH_2 \quad (II),$$

are heated under reflux in a solvent until the imidazoline compound has formed.

**3.** Process according to claim 1 or 2, characterised in that a molar ratio of methyl salicylate to amine of 3:1 to 4:1 is used.

## Revendications

### Revendications pour les Etats contractants suivants : AT, CH, DE, FR, GB, IT, LI, NL, SE

1. Dérivés de l'imidazoline de formule I

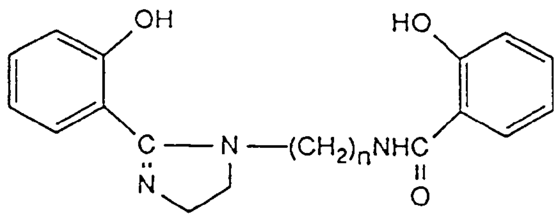

(I)

dans laquelle n vaut 2 ou 3.

2. Procédé pour préparer des dérivés de l'imidazoline de formule I selon la revendication 1, caractérisé en ce qu'on chauffe du salicylate de méthyle avec une amine de formule II

$$NH_2CH_2CH_2NH(CH_2)_nCH_2 \qquad (II)$$

dans laquelle n vaut 2 ou 3, ce qui permet d'obtenir un composé de formule III

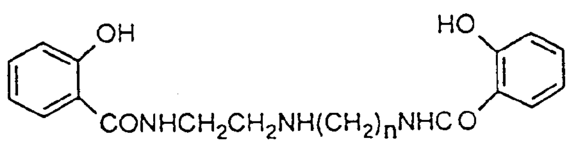

(III)

puis on chauffe le composé de formule III à une température plus élevée, une élimination de l'eau et une cyclisation permettant d'obtenir un dérivé de l'imidazoline de formule I.

3. Procédé pour préparer des dérivés de l'imidazoline selon la revendication 2, caractérisé en ce qu'on chauffe du salicylate de méthyle et une amine de formule II

$$NH_2CH_2CH_2NH(CH_2)_nCH_2 \qquad (II)$$

dans un solvant au reflux jusqu'à formation du dérivé de l'imidazoline.

4. Procédé selon la revendication 2 ou 3, caractérisé en ce qu'on utilise une proportion molaire du salicylate d'éthyle à l'amine de 3:1 à 4:1.

5. Mélange durcissable contenant (A) une résine époxyde, et (B) en tant que durcisseur latent pour (A), du dicyanodiamide ou un hydrazide d'un acide polycarboxylique, et (C) en tant qu'accélérateur de durcissement, un dérivé de l'imidazoline selon la revendication 1, dispersé sous forme de poudre dans le mélange.

6. Mélange selon la revendication 5, contenant une résine époxyde (A) comportant en moyenne plus d'un groupe glycidyle dans sa molécule, groupe lié à un atome d'oxygène, d'azote ou de soufre.

7. Mélange selon la revendication 6, dans lequel la résine époxyde (A) est liquide et est un éther polyglycidylique, un ester polyglycidylique, une N,N'-diglycidylhydantoïne ou un composé poly(N-glycidylique) d'une amine aromatique.

8. Mélange selon la revendication 5, dans lequel l'accélérateur de durcissement (C) est présent en des quantités de 0,1 à 20 % en poids par rapport à la résine époxyde (A).

9. Mélange selon la revendication 5, dans lequel l'accélérateur de durcissement (C) est présent en des quantités de 1 à 10 % en poids par rapport à la résine époxyde (A).

10. Mélange selon la revendication 5, contenant en outre, en tant que modifiant choc, un polymère diénique élastomère.

**11.** Utilisation du mélange durcissable selon la revendication 5 pour coller ou sceller deux surfaces l'une à l'autre, en particulier des surfaces métalliques.

**Revendications pour l'Etat contractant suivant : ES**

**1.** Procédé pour préparer des dérivés de l'imidazoline de formule I

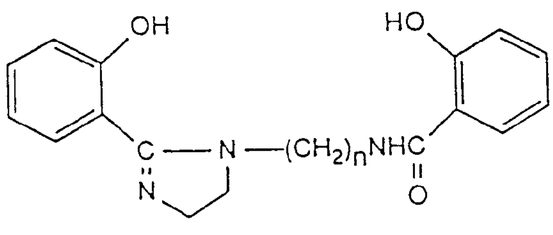

(I)

dans laquelle n vaut 2 ou 3, caractérisé en ce qu'on chauffe du salicylate de méthyle avec une amine de formule II

$NH_2CH_2CH_2NH(CH_2)_nCH_2$ (II)

dans laquelle n vaut 2 ou 3, ce qui permet d'obtenir un composé de formule III

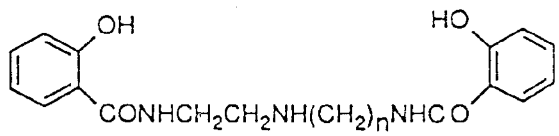

(III)

puis on chauffe le composé de formule III à une température plus élevée, une élimination de l'eau et une cyclisation permettant d'obtenir un dérivé de l'imidazoline de formule I.

**2.** Procédé pour préparer des dérivés de l'imidazoline selon la revendication 1, caractérisé en ce qu'on chauffe du salicylate de méthyle et une amine de formule II

$NH_2CH_2CH_2NH(CH_2)_nCH_2$ (II)

dans un solvant au reflux jusqu'à formation du dérivé de l'imidazoline.

**3.** Procédé selon la revendication 1 ou 2, caractérisé en ce qu'on utilise une proportion molaire du salicylate de méthyle à l'amine de 3:1 à 4:1.